# EUROPEAN PATENT APPLICATION

(11) **EP 1 254 896 A1**
(43) Date of publication of application: **06.11.2002**
(21) Application number: 01201612.7
(22) Date of filing: 03.05.2001
(51) Int. Cl.: C07D 213/20, C07D 213/79, C07D 213/16, A61K 48/00

(54) **Cationic amphiphiles, preparation and method for transfection**

(71) Applicant: Applied NanoSystems B.V., 9700 AC Groningen (NL)
(72) Inventor: Hulst, Anthony Jacques Ronald Lambert, 9722 JK Groningen (NL); Smisterova, Jarmila, 9753 BL Haren (NL); Engberts, Jan Bernard Frederik Nicolaas, 9742 EC Groningen (NL); Hoekstra, Dirk, 9801 GJ Zuidhorn (NL)
(74) Representative: Prins, Adrianus Willem

(57) **Abstract**

The invention relates to novel cationic amphiphiles having an aromatic ring comprising at least one nitrogen atom. The cationic amphiphiles are particularly useful for the introduction of biologically active compounds such as DNA, RNA, proteins and the like into eukaryotic cells. The cationic amphiphiles are represented by the following basic formula:

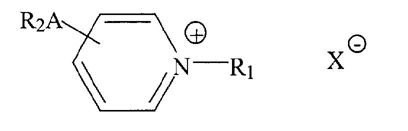

Characteristic is that R1 and R2 are single C6-C24 carbon chains. For the mode of action it is assumed that R1 folds back over the aromatic ring towards R2.

## Description

### FIELD OF THE INVENTION

The invention relates to novel cationic amphiphiles having an aromatic ring comprising a nitrogen atom, particularly useful for the introduction of biologically active compounds such as DNA, RNA, proteins and the like into eukaryotic cells. Also the invention relates to a method for the preparation of such cationic amphiphiles. Further the invention relates to a method for the introduction of polynucleotides into eukaryotic cells. In particular the introduction of DNA, RNA or (antisense-)oligonuceotides is of interest for which usually the term transfection is used.

### BACKGROUND OF THE INVENTION

The introduction of biologically active compounds such as DNA, RNA, proteins and the like into eukaryotic cells is of interest in the light of several applications. One of the most important applications in which biologically active compounds are introduced into a cell is transfection of the cell with DNA or RNA. Transfection allows the study of the particular function of the DNA or RNA that is introduced. In particular with respect to the introduction of genes the function and properties of the corresponding protein products are of interest. Also transfection can be used to genetically modify cells into which the DNA or RNA is introduced. For instance DNA can be introduced into a cell that does not normally contain such DNA and thus not normally expresses the corresponding protein encoded by the DNA. For example the expression of a marker protein encoded by the introduced DNA can be achieved in a cell. In addition, if the cell is a micro-organism the production of a protein of interest by such a micro-organism could be achieved upon introduction of exogenic DNA in a suitable form in the micro-organism. Subsequently, the protein of interest can be harvested from the micro-organism. A protein of interest can be a protein having for instance therapeutic purposes.

Transfection can also be used for therapeutic purposes as such. For example, antisense-oligonucleotides can, upon introduction into a cell, bind to complementary single stranded nucleic acid molecules or even to double stranded nucleic acid molecules by appropriate base pairing. In doing so the function of the single or double stranded nucleic acid can be disrupted. In addition transfection can be used in gene therapy. Genes or parts of genes can be introduced to replace or repair defect genes or even introduce missing genes thereby offering the possibility of curing a disease at the source.

Also proteins and other macromolecules such as, for instance, carbohydrates are introduced into cells for therapeutic and screening purposes. For example, immunisation is enhanced by introducing an immunogenic protein into a cell, so that it is more efficiently processed and presented on the surface of the cells, thereby enhancing the immunogenic response. Also, negatively charged macromolecules which have a function in a cell are transported past the cell membrane into the cytoplasm where they exert their effect. Factors which enhance or hinder transcription of DNA can be used in a screening test to verify the transcription of a gene of interest. Such transcription assays can be used for screening compounds to determine their effect against a particular cellular macromolecule, for example, a cell receptor.

Various ways of introducing biologically active compounds into cells exist. To date much effort is directed at improving on existing ways and developing new methods and systems for transfection. This invention particularly concerns the use of non-viral systems for the introduction of biologically active compounds, more specifically it concerns cationic amphiphiles. Since the introduction of the quaternary ammonium containing amphiphile dioleoyloxypropyl trimethyl ammonium chloride by Felgner et al. (Proc. Natl. Acad. Sci USA, 1987, 84, 7413-7417), which in combination with the phospholipid dioleoylphosphatidylethanolamine (DOPE) is commercially available as Lipofectin™, many more cationic amphiphiles have been developed and marketed. For example, cationic amphiphiles having a pyridinium group, which is an aromatic ring comprising a nitrogen atom, as cationic part for introducing biologically active compounds into eukaryotic cells have been developed and are disclosed in EP 0755924.

However, despite important progress in the formulation of non-viral gene delivery systems, there remains a need for more efficient systems, since the transfection efficiency of non-viral systems including synthetic cationic amphiphiles is usually below that of viral vectors. Furthermore, still many problems arise *in vivo* and *in vitro* hampering the realization of high and reproducible levels of transfection. This is for instance due in part to the poor stability of the non-viral systems in biological fluids and culture media.

Many known compounds used for transfection experiments are toxic for eukaryotic cells. In addition problems may occur in scaling up where the conditioned medium reduces the effect of known transfecting agents.

### SUMMARY OF THE INVENTION

The aim of the present invention is to provide novel compounds that can serve as an alternative for existing cationic amphiphiles for the introduction of biologically active compounds into eukaryotic cells.

It is an object of the invention to improve on the properties of known transfection agents. Such improvement can be achieved for instance with respect to efficiency of transfection, toxicity of the transfection system, specificity of the transfection system, stability of the transfection system, method of transfection and use of the transfection system.

The aim of the invention is achieved with a cationic amphiphile having an aromatic ring comprising a nitrogen atom according to the following formula 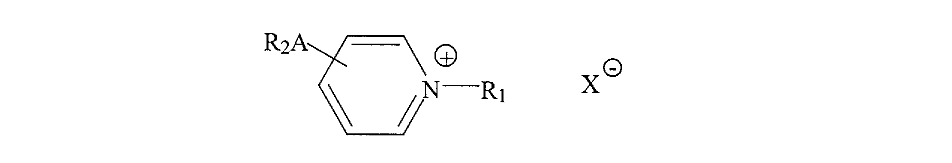 in which:
R1 is selected from the group consisting of:
   a branched or linear (C6-C24) carbon chain optionally interrupted by one or more heteroatoms, optionally containing one or more functional groups, optionally containing one or more double or triple carbon-carbon bonds or combinations of double and triple carbon-carbon bonds, optionally being substituted or combinations thereof and
R3-Ar-A-R2 in which R3 is a C2-C10 carbon chain, Ar is an aromatic or a heteroaromatic ring optionally to which relative to R3 in the ortho-, meta-, or para-position A-R2 is attached,
A is attached to the aromatic ring in the ortho-, meta- or para-position relative to the nitrogen atom in the aromatic ring,
A is selected from the group consisting of CH₂, O, S, ,S-S, NH, OC=O, O=CO, SC=O, O=CS, NHC=O, O=CNH, CH=N, N=CH, phosphate, alkylphosphate and phosphonate R2 represents a branched or linear (C6-C24) carbon chain optionally interrupted by one or more heteroatoms, optionally containing one or more functional groups, optionally containing one or more double or triple carbon-carbon bonds or combinations of double and triple carbon-carbon bonds, optionally being substituted or combinations thereof X⁻ is a physiologically acceptable anion.

### DESCRIPTION OF THE INVENTION

The cationic amphiphiles according to the invention comprise of an aromatic ring to which two carbon chains (R1 and R2) are attached. The aromatic ring comprises a nitrogen atom. One of the two carbon chains (R1) is attached to the nitrogen atom in the ring. The second carbon chain (R2) is attached to the ortho-, meta- or para-position relative to this nitrogen. Both groups R1 and R2 in the formula can be identical but this is not necessary. In claims 2-7 preferred subclasses of the cationic amphiphiles according to the invention are given. In a particular embodiment of the cationic amphiphiles according to the invention R1 is longer than R2.

In their most elementary form the carbon chains R1 and R2 are linear alkyl chains of 6-24 carbon atoms. Optionally one of the two or both carbon chains comprise one or more unsaturations in the form of double or triple carbon-carbon bonds. Also it is possible that the carbon chains comprise, optionally in combination with one or more unsaturations, one or more heteroatoms in the chain and/or one or more functional groups in the chain and/or substitutions on the chain. It is also possible that the carbon chain is branched. Preferably such branching does not occur on the first six carbon atoms calculated starting from the aromatic ring. Such branching can occur in combination with the presence of unsaturated carbon-carbon bonds and also in combination with the presence of heteroatoms in the chain and/or with the presence of functional groups and/or in the presence of substitutions.

In one embodiment the carbon chain R1 on the aromatic nitrogen atom comprises an aromatic group. In particular the aromatic group can be a phenyl group. The aromatic group, represented by Ar, in R1 is positioned near the nitrogen atom containing the aromatic ring. Near in this respect means that the carbon chain connecting the nitrogen containing aromatic ring and the aromatic group in R1 is of such a length that "backfolding" of R1 towards the nitrogen atom containing aromatic ring allows alignment of the aromatic group in R1 with the nitrogen atom containing aromatic ring. With the aid of models the skilled person will be able to determine an appropriate length of the chain connecting Ar and the nitrogen containing aromatic ring for backfolding to occur. Backfolding resulting in sufficient alignment is achieved with R3 which is a C2-C10 carbon chain. Optionally attached to the aromatic group in R1 is A-R2 in the ortho-, meta- or para-position relative to R3, in which R2 is defined as above and A as will follow. Another particular embodiment is a cationic amphiphile in which Ar is a heteroaromatic ring. A heteroaromatic ring is an aromatic ring comprising one or more heteroatoms such as O, N and S. In particular Ar is a heteroaromtaic ring which comprises a nitrogen atom. R3 is attached to the nitrogen in this aromatic ring Ar. In the ortho-, meta- or para-position in the aromatic ring A-R2 is attached.

R1 is directly attached to the nitrogen atom in the aromatic ring. R2 is attached to a carbon atom in the aromatic ring via group A. Various ways exist for attaching R2 to an aromatic carbon atom. The skilled person will be able to determine what groups are available for A. Particularly suited as group A for attachment is a group chosen from CH₂, O, S, S-S, NH, OC=O, O=CO, SC=O, O=CS, NHC=O, O=CNH, CH=N, N=CH, phosphate, alkylphosphate and phosphonate.

X⁻ represents a physiologically acceptable anion. The cationic amphiphiles of the invention can be used for *in vitro* as well as *in vivo* purposes. In this respect it may vary what anions are physiologically acceptable. The skilled person will be able to determine for what purpose which anion may be suitable. Examples of suitable anions are Cl⁻, Br⁻, I⁻, HSO₄⁻, H₂PO₄⁻, ClO₄⁻ and organic anions such as CH₃CO₂⁻, ⁻O₂CCO₂⁻ and the like.

As mentioned above the R1 and R2 groups in the cationic amphiphiles of the invention may contain one or more unsaturated carbon-carbon bonds. Also the R1 and R2 groups may contain, in any position, one or more heteroatoms such as O, N and S. Such a heteroatom can be part of a functional group. Thus R1 and R2 may contain, in any position one or more functional groups such as ethers, disulphides, esters, amides, phosphates, imines, amidines and the like. For research or diagnostic or therapeutic purposes R1 or R2 or both may also comprise fluorescent groups, such as fluorescein rhodamine, acridine, diphenylhexatrienepropionic acid and the like in the chain or as substituent attached to the chain or, R1 and/or R2 may comprise or be substituted with radioactive labels. Of particular interest are substituents that can be involved in targeting of cells. For instance ligands for particular receptors on cells or antibodies or parts of antibodies comprising binding domains for a particular epitope at or in the neighbourhood of the site where the incorporated biologically active compound has to exert its activity can be attached to R1 and/or R2. Such targeting substituents can be attached directly or for instance through a spacer. Also functional groups and/or substituents that can be involved in the release from endosomes in cells such as pH labile groups or substituents can be of interest.

Optionally the carbon chains in the vicinity of the nitrogen containing aromatic ring are substituted with groups introducing additional positive charge such as for instance amino groups that are protonated under physiological conditions and trialkylammonium groups. In this respect also substituents which can be involved in hydrogen bonding are considered.

The cationic amphiphiles according to the invention can be synthesised following known procedures such as described in EP 0755924 and as will be further illustrated in the examples. In short 4-methylpyridine is treated with base and subsequently mono-alkylated on the 4-methyl group to introduce R2. Next the nitrogen in the pyridine ring is quaternized with an alkyl halide to introduce R1 followed by ion-exchange to obtain the desired X⁻ as counter-ion. However, in order to obtain the desired compounds in acceptable yield and purity the general known method described above is not always satisfactory. Surprisingly, it has been found that cationic amphiphiles that would not be available in an economic acceptable manner using known procedures can be synthesised by applying the microwave technique in the procedure. In particular the attachment of R1 to the nitrogen in the aromatic ring is carried out under microwave conditions. In general this means that the step of attaching a group on the nitrogen in the nitrogen atom containing aromatic ring is carried out in a microwave oven. The attachment reaction is particularly substitution of a halide in the group to be attached. Depending on the specific reaction that is carried out the skilled person will be able to determine the optimal reaction time and settings for the microwave oven. For several cationic amphiphiles according to the invention the reaction time and settings are given in detail in the examples.

Thus the invention also relates to a method for the preparation of cationic amphiphiles having a nitrogen atom containing aromatic ring and a group attached to said nitrogen atom in which the step of introducing a group on the nitrogen atom is carried out in a microwave oven. In particular this method is used for the introduction of R1 onto the nitrogen in the aromatic ring in the formula in claim 1.

In a further aspect the invention also relates to a method for the introduction of biologically active compounds into eukaryotic cells. A biologically active compound can be any compound capable of forming for instance a complex with the cationic amphiphiles of the invention. Preferably such a biologically active compound contains at least one negative charge per molecule. Examples of such compounds are DNA, RNA, proteins, carbohydrates and the like and combinations thereof. The introduction into cells is carried out following known protocols as set out in the examples. In short an appropriate amount of cationic amphiphile, usually but not necessarily in combination with a helper lipid such as DOPE, is mixed with an appropriate amount of for instance DNA. The resulting complex is brought into contact with cells. The appropriate amount of cationic amphiphile of the formula for a given amount of biologically active compound depends on their respective charges. The +/-charge ratio between the cationic amphiphile and the molecule to be introduced into cells generally varies between 0.1-10, preferably between 0.5-5. The optimal ratio depends on the specific amphiphile, biologically active compound and cells used and is readily ascertained by the skilled person.

Thus teh invention also relates to a method for the introduction of biologically active compounds into eukaryotic cells said method comprising the steps of mixing a cationic amphiphile according to th einvention with a biologically active compound and bringing the mixture thus obtained into contact with the cells to be transfected.

With respect to diagnostic and therapeutic purposes in particular vertebrate eukaryotic cells, preferably mammalian eukaryotic cells are of interest. Also eukaryotic cell lines used in tissue culture techniques are suited. Such cell lines can be adherent or suspension cell lines.

In one specific embodiment the method for the introduction of biologically active compounds into eukaryotic cells yields surprisingly good results. In particular for cationic amphiphiles having an aromatic ring comprising at least one nitrogen atom, transfection is achieved if the cationic amphiphile, in the absence of a helper lipid, is complexed with DNA in a small volume of saline buffer, and then the resulting complex is diluted in the appropriate medium for transfection. More in particular efficiency of transfection is increased if the cationic amphiphile comprises additional positive charge in the vicinity of the nitrogen atom containing aromatic ring. This method is particularly suited for the introduction of polynucleotides such as DNA, RNA or (antisense-)oligonuceotides. As additional positive charge, in particular the presence of at least one ammonium group is effective. A suitable position for the additional positive charge is in the carbon chain that is attached to the nitrogen in the aromatic ring. In the vicinity of the aromatic ring in this respect means that the positive charge is present in, or present in a substituent on, the first 10 carbon atoms in the carbon chain calculated from nitrogen atom in the aromatic ring. A small volume of buffer means such a volume which has to be diluted at least 5 times, preferably at least 10 times to achieve an appropriate concentration for contacting the DNA/cationic amphiphile complex with cells. Saline buffer is for instance HEPES Buffered Saline (HBS, 10 mM HEPES, 150 mM NaCl, pH 7.4). Medium is the appropriate culture medium for the particular cells to be transfected.

Thus the invention also relates to a method for the introduction of biologically active compounds into eukaryotic cells comprising the steps of first mixing a cationic amphiphile having an aromatic ring comprising at least one nitrogen atom in the absence of helper lipid with a biologically active compound in a small volume of saline buffer and then diluting the resulting complex in medium and bringing the mixture thus obtained into contact with the cells to be transfected. Preferably the biologically active compound is DNA, RNA or an (antisense-)oligonuceotide. Preferably the cationic amphiphile comprises additional positive charge in the vicinity of the nitrogen atom containing aromatic ring, in particular the cationic amphiphile comprises one or more ammonium groups.

The invention also relates to a composition of a cationic amphiphile according to the invention and a biologically active compound, optionally in combination with a pharmaceutically acceptable carrier. In particular the biologically active compound is DNA, RNA or an (antisense-)oligonucleotide.

Using the cationic amphiphiles of the invention it is possible to transfect a variety of cells with no signs of toxicity. The possible mode of action of the cationic amphiphiles according to the invention is based on the cooperation of the two carbon chains that are on both sides of the nitrogen containing aromatic ring. Backfolding over/across the aromatic ring has to occur to match the two carbon chains and allow formation of a lamellar structure or even formation of a bilayer. From NOE NMR data it can be concluded that such backfolding indeed can occur. It is assumed that the capability of amphiphiles to form a bilayer structure is favourable for transfection. Such a lamellar arrangement results in a beneficial complex formation with DNA. Upon interaction with a cell, in particular the phospholipid bilayer of the cell membrane, the amphiphile/DNA complex is destabilized by unfolding of a carbon chain whereby presumably the lamellar structure of the amphiphiles is disrupted. Not only allows this the release of the DNA but also insertion of the unfolded chain in the cell membrane might destabilize the membrane allowing the introduction of the DNA into the cell.

### EXAMPLES

### Preparation of 1-(9'-cis-octadecenyl)-4-(10"-cis-nonadecenyl) pyridinium Chloride

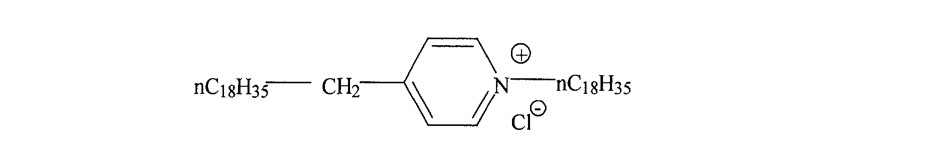

### 4-(10'-cis)-nonadecenyl pyridine

A solution of 5.05 g (49.9 mmol) diisopropylamine in 300 ml of dry THF was cooled to 0° C under a nitrogen atmosphere. Then, 31.3 ml of a n-butyl lithium/hexane solution (1.6 M in hexane's, 50.0 mmol) was added dropwise and the resulting reaction mixture was stirred for an additional 30 min at 0° C. Subsequently, 4.50 g of 4-picoline (48.5 mmol) was added dropwise at -50° C under careful temperature control. After completion, the orange-yellow solution was stirred for another 30 min at -50° C. Hereafter, 18.33 g of 1-Iodo-(9-cis)-octadecene (85% cis, 48.5 mmol) in 25 ml of dry THF was added dropwise at -50° C under careful temperature control. The bright red solution was stirred for an additional 60 min at -50° C and at RT overnight. The reaction was quenched by adding 300 ml of a saturated NH₄Cl solution. The organic layer was separated and the waterlayer extracted with three portions of ether (50 ml). The combined organic fractions were washed twice with H₂O (100 ml) and brine (200 ml), dried over Na₂SO₄, filtered and concentrated in vacuo to yield a viscous brown oil. The product was purified over a column of 300 g neutral Al₂O₃ (act. III, freshly prepared) using n-hexane/diethyl ether as gradient eluent mixture (100:0 > 80:20) yielding 4-(10'-cis)-nonadecenyl pyridine as a colourless viscous oil. Yield after purification 12.20 g, 36.9 mmol, 76%. The cis:trans ratio at the double bonds was unchanged 85:15 based upon ¹H NMR integration of the protons at δ 5.25 and 5.33 ppm, which are broad singlets when selectively homo nuclear decoupled at ∼ δ 1.90 ppm.
¹H NMR (CDCl₃): δ 0.81 (t, ³J= 6.95 Hz, 3H), 1.15-1.35 (br, m, 26H), 1.52-1.58 (br, m, 2H), 1.82-1.98 (br, m, 4H), 2.52 (t, ³J= 7.69 Hz, 2H), 5.21-5.38 (m, 2H), 7.02 (d, ³J_{AB}= 5.49 Hz, 2H), 8.41 (d, ³J_{AB}= 5.49 Hz, 2H).
¹³C NMR (CDCl₃): δ 12.60, 21.17, 21.78, 24.09, 25.69, 27.68, 27.75, 27.81, 27.99, 28.25, 28.79, 30.39, 31.09, 66.44, 122.36, 123.09, 128.28, 128.41, 128.76, 148.08.
Elemental analysis for C₂₄H₄₁N (343.60) calcd. C 83.90%, H 12.03%, N 4.08%; found C 83.87%, H 12.07%, N 4.04%. EI-MS calcd. for C₂₄H₄₁N 343.3 found 343.3.

### 1-(9'-cis-octadecenyl)-4-(10''-cis-nonadecenyl) pyridinium Iodide

A pre-mixed (1 min ultrasonic bath) amount of 4-(10'-cis)-nonadecenyl pyridine (0.50 g, 1.51 mmol) and 1-Iodo-(9-cis)-octadecene (85% cis, 0.75 g, 1.98 mmol) was placed in a MicroWave oven and carefully deoxygenated. The mixture was reacted using Pulsed Intervals [10 min 600 Watt (90%) followed by 10 min of 300 Watt (50%), sequence repeated three times], resulting in a deep red-brown very viscous oil. Based upon ¹H NMR, conversion had evolved to approximately 90%. The material was purified over a column of 50 g neutral Al₂O₃ (act. III, freshly prepared) using n-hexane as eluent to remove impurities and (excess) starting materials. The material was brought on the column dissolved in a minimum amount of CHCl₃. After changing the solvent to n-hexane/methanol (98:2), 1-(9-cis-octadecenyl)-4-(10'-cis-nonadecenyl) pyridinium iodide was obtained as a colourless viscous oil that colours quickly. The material solidifies completely if stored at temperatures below 4° C. Yield 0.83 g, 1.17 mmol, 78%. The cis:trans ratio at the double bonds was unchanged 85:15 based upon ¹H NMR integration of the protons at δ 5.32 and 5.40 ppm, which are broad singlets when selectively homo nuclear decoupled at ∼ δ 2.00 ppm.
¹H NMR (CDCl₃): δ 0.85 (t, ³J= 6.8 Hz, 6H), 1.20-1.45 (br, m, 46H), 1.50-1.80 (br, m, 4H), 1.90-2.10 (br, m, 8H), 2.87 (t, ³J= 8.12 Hz, 2H), 4.87 (t, ³J= 7.31 Hz, 2H), 5.28-5.45 (m, 4H), 7.83 (d, ³J_{AB}= 6.59 Hz, 2H), 9.23 (d, ³J_{AB}= 6.59 Hz, 2H).
¹³C NMR (CDCl₃): δ 12.60, 21.25, 24.50, 25.68, 27.52, 27.73, 27.76-27.78 (several), 27.87, 28.09, 28,18, 28.23, 30.28, 30.37, 31.08, 34.47, 59.86, 126.40, 128.12, 128.23, 128.44, 128.50, 142.62, 161.81.
Elemental analysis for C₄₂H₇₆NI (721.98) calcd. C 69.87%, H 10.61%, N 1.94%, I 17.58%; found C 69.74%, H 10.53%, N 1.98%, I 17.55%. EI-MS calcd. for C₄₂H₇₆NI 721.5, found 721.3.

### 1-(9'-cis-octadecenyl)-4-(10''-cis-nonadecenyl) pyridinium Chloride

0.50 g (0.70 mmol) of 1-(9'-cis-octadecenyl)-4-(10"-cis-nonadecenyl) pyridinium iodide (purified) was dissolved in 5 ml of methanol and this solution was eluted over a DEAE Sephadex A-25 column (50 g, Chloride form) yielding 1-(9'-cis-octadecenyl)-4-(10"-cis-nonadecenyl) pyridinium chloride (0.40 g, 0.66 mmol, 94%) as a colourless viscous oil. The material solidifies completely if stored at temperatures below 4° C. The cis:trans ratio at the double bonds was unchanged 85:15 based upon ¹H NMR integration of the protons at δ 5.30 and 5.38 ppm, which are broad singlets when selectively homo-nuclear decoupled at ∼ δ 2.00 ppm.
¹H NMR (CDCl₃): δ 0.85 (t, ³J= 6.8 Hz, 6H), 1.20-1.45 (br, m, 46H), 1.50-1.80 (br, m, 4H), 1.90-2.10 (br, m, 8H), 2.87 (t, ³J= 8.1 Hz, 2H), 4.98 (t, ³J= 7.3Hz, 2H), 5.32-5.54 (m, 4H), 7.94 (d, ³J_{AB}= 6.22 Hz, 2H), 9.43 (d, ³J_{AB}= 6.22 Hz, 2H).
¹³C NMR (CDCl₃): δ 12.60, 21.25, 24.50, 25.68, 27.52, 27.73, 27.76-27.78 (several), 27.87, 28.09, 28,18, 28.23, 30.28, 30.37, 31.08, 34.47, 59.96, 126.40, 128.12, 128.23, 128.44, 128.50, 142.65, 161.92.

Elemental analysis for C₄₂H₇₆NCl (630.53) calcd. C 80.01%, H 12.15%, N 2.22%, Cl 5.62%; found C 79.85%, H 12.08%, N 2.27%, Cl 5.58%. EI-MS calcd. for C₄₂H₇₆NCl 629.6, found 629.6.

### 1-(6"-Phenylhexyl)-4-(10'-cis-nonadecenyl) pyridinium Bromide

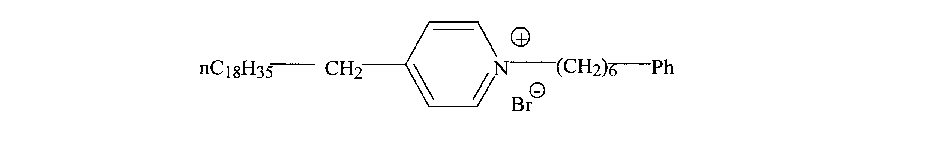

### 1-(6''-Phenylhexyl)-4-(10'-cis-nonadecenyl) pyridinium Bromide

A pre-mixed (1 min ultrasonic bath) amount of 0.50 g (1.46 mmol) 4-(10'-cis)-nonadecenyl pyridine and 1.67 g 1-bromo-6-phenylhexane (7.30 mmol) was placed in a Micro Wave oven and carefully deoxygenated. The mixture was reacted using Pulsed Intervals [10 min 600 Watt (90%) followed by 10 min of 300 Watt (50%), sequence repeated three times], resulting in a deep brown material. The crude product mixture was purified by column chromatography (act. III, freshly prepared) using diethyl ether as eluent to remove impurities and (excess) starting materials. The material was brought on the column dissolved in a minimum amount of CHCl₃. After changing the solvent to diethyl ether/methanol (98:2) the product was obtained as a slightly coloured viscous oil. Yield 0.73 g, 1.26 mmol, 86%.
¹H NMR (CD₃OD): δ 0.81 (t, ³J= 6.02 Hz, 3H), 1.13-1.40 (br, m, 30H), 1.56 (t, ³J= 8.02 Hz, 2H), 1.64 (t, 3J= 7.04 Hz, 2H), 1.84-2.04 (br, m, 4H), 2.52 (t, ³J= 8.02 Hz, 2H), 2.86 (t, ³J= 7.94 Hz, 2H), 4.46 (t, ³J= 6.68 Hz, 2H), 5.22-5.36 (m, 2H), 7.01-7.18 (m, 5H), 7.86 (d, ³J_{AB}= 7.02 Hz, 2H), 8.73 (d, ³J_{AB}= 7.02 Hz).

### 1-Methyl-4-(-carbo-(9'-cis-nonadecenyloxy)) pyridinium Chloride

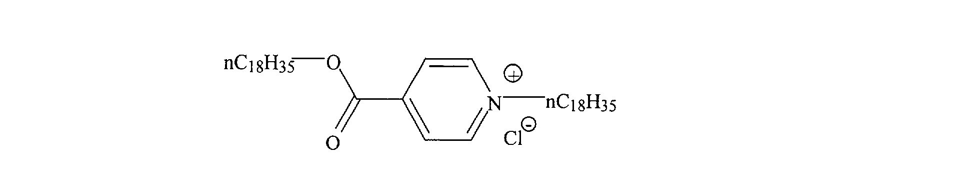

### 4-carbo-(9'-cis-nonadecenyloxy) pyridine

A solution of 2.0 g isonicotinoylchloride hydrochloride (11.24 mmol) and triethylamine (2.53 g, 25 mmol) in 50 ml of CH₂Cl₂ was cooled to 0° C. 1-hydroxy-(9-cis)-octadecene (3.01 g, 11.24 mmol, 85% cis) dissolved in 25 ml of CH₂Cl₂ was added dropwise and the reaction was allowed to react for 60 min. Subsequently, the reaction mixture was taken to reflux overnight. The mixture was cooled to 0° C and filtered, the filtrate was concentrated at reduced pressure yielding a yellow viscous oil. The crude product mixture was purified by column chromatography (act. III, freshly prepared, 75 g) using hexane as eluent to remove impurities and (excess) starting materials. The material was brought on the column dissolved in a minimum amount of CHCl₃. Changing the solvent to hexane:CHCl₃ (gradient, 100:0 to 80:20 and a few drops of triethylamine) afforded the product as a colourless oil that partly solidifies upon standing. Yield 2.09 g, 5.12 mmol, 45%. The cis:trans ratio at the double bonds was unchanged 85:15 based upon ¹H NMR integration of the protons at δ 5.27 and 5.33 ppm, which are broad singlets when selectively homo nuclear decoupled at ∼ δ 1.95 ppm.
¹H NMR (CDCl₃): δ 0.80 (t, ³J= 5.86 Hz, 3H), 1.15-1.42 (br, m, 22H), 1.71 (m, 2H), 1.87-2.01 (br, m, 4H), 4.28 (t, 3J= 6.65 Hz, 2H), 5.22-5.38 (br, m, 2H), 7.77 (d, ³J_{AB}= 5.86 Hz, 2H), 8.79 (d, ³J_{AB}= 5.86 Hz, 2H).
¹³C NMR (CDCl₃): δ 13.98, 22.56, 25.84, 27.04, 27.09, 28.49, 29.10, 29.19, 29.27, 29.40, 29.58, 29.63, 31.78, 32.49, 65.82, 122.70, 129.60, 129.89, 137.52, 150.44, 165.02.

### 1-(9 '-cis-nonadecenyl)-4-(-carbo-(9''-cis-nonadecenyloxy)) pyridinium Iodide

0.40 g 4-carbo-(9'-cis-nonadecenyloxy) pyridine (0.97 mmol) and 0.57 g 1-Iodo-(9-cis)-octadecene (85% cis, 1.50 mmol) were placed in a MicroWave oven and carefully deoxygenated. The mixture was reacted under continuous progress control (TLC) using Pulsed Intervals [10 min 600 Watt (50%) followed by 10 min of 300 Watt (15%)] until the conversion had evolved to approx. 85%. This process takes about 80 minutes, resulting in a reddish very viscous material. The crude product mixture was purified by column chromatography (act. III, freshly prepared, 25 g) using hexane as eluent to remove impurities and (excess) starting materials. The material was brought on the column dissolved in a minimum amount of CHCl₃. Changing the solvent to hexane:CHCl₃ (gradient, 100:0 to 80:20) and eventually to hexane:CHCl₃:methanol (75:20:5) afforded the product as a colourless oil, that partly solidifies upon standing. Yield 0.54 g, 0.69 mmol, 71%.The material was used immediately for ion exchange due to the relative low stability as Iodide salt. The cis:trans ratio at the double bonds was unchanged 85:15 based upon ¹H NMR integration of the protons at δ 5.25 and 5.31 ppm, which are broad singlets when selectively homo nuclear decoupled at ∼ δ 1.90 ppm.
¹H NMR (CDCl₃): δ 0.82 (dt, ³J= 6.96 Hz, 6H), 1.20-1.45 (br, m, 22H), 1.48-1.54 (br, m, 20H), 1.72-1.83 (m, 2H), 1.90-2.08 (br, m, 12 H), 4.40 (t, ³J= 6.95 Hz, 2H), 4.98 (br, t, ³J= 7.32 Hz, 2H), 5.20-5.35 (m, 4H), 8.48 (d, ³J_{AB}= 5.86 Hz, 2H), 9.41 (d, ³J_{AB}= 5.86 Hz, 2H).

### 1-(9 '-cis-nonadecenyl)-4-(-carbo-(9''-cis-nonadecenyloxy)) pyridinium Chloride

A solution of 0.25 g 1-(9'-cis-nonadecenyl)-4-(-carbo-(9''-cis-nonadecenyloxy)) pyridinium Iodide (0.32 mmol) and 0.46 g AgCl (3.2 mmol) in 25 ml of dry acetone was placed under a nitrogen atmosphere and taken to reflux. During reflux the solution de-coloured from yellow to white. The reaction mixture was cooled to approx. 0° C and filtered. The residue was washed with small amounts of dry acetone. The combined acetone layers were combined and concentrated in vacuo. The crude product mixture was purified by column chromatography (act. III, freshly prepared, 25 g) using hexane:CHCl₃ (85:15) to remove impurities. Changing the solvent to hexane:CHCl₃:methanol (75:20:5) afforded the product as a colourless oil/gel, that partly solidifies upon standing. Yield 0.16 g, 0.25 mmol, 78%. The cis:trans ratio at the double bonds was unchanged 85:15 based upon ¹H NMR integration of the protons at δ 5.25 and 5.30 ppm, which are broad singlets when selectively homo nuclear decoupled at ∼δ 1.90 ppm.
¹H NMR (CDCl₃): δ 0.82 (dt, ³J= 6.96 Hz, 6H), 1.20-1.45 (br, m, 22H), 1.48-1.54 (br, m, 20H), 1.72-1.83 (m, 2H), 1.90-2.08 (br, m, 12 H), 4.67 (t, ³J= 6.87 Hz, 2H), 5.09 (br, t, ³J= 7.24 Hz, 2H), 5.20-5.45 (m, 4H), 8.58 (d, ³J_{AB}= 5.81 Hz, 2H), 9.61 ((d, ³J_{AB}= 5.81 Hz, 2H).

According to the experimental procedures given above the following compounds have been synthesized:

### 1-Benzyl-4-nonadecyl pyridinium Chloride

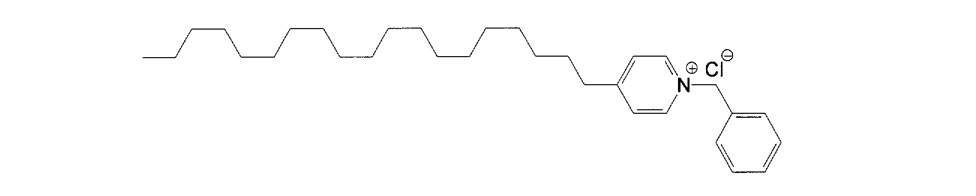

¹H NMR (CD₃OD): δ 0.81 (t, ³J= 7.00 Hz, 3H), 1.13-1.35 (m, 32 H), 1.58-1.71 (m, 2H), 2.86 (t, ³J= 7.00 Hz, 2H), 5.68 (br, m, 2H), 7.34 (br, m, 5H), 7.87 (d, ³J_{AB}= 6.03 Hz, 2H), 8.79 (d, ³J_{AB}= 6.03 Hz, 2H).

### 1-Benzyl-4-(cis-10')-nonadecenyl pyridinium Chloride

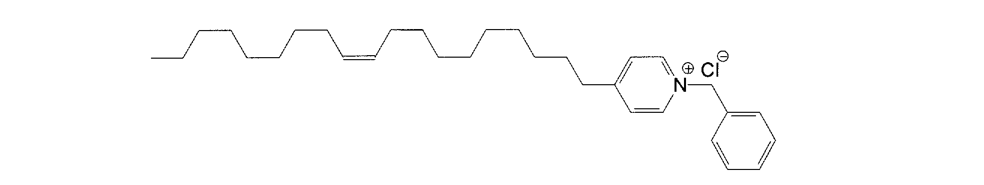

¹H NMR (CDCl₃): δ 0.79 (t, ³J= 7.01 Hz, 3H), 1.09-1.36 (m, 24H), 1.55 (q, ³J= 7.01 Hz, 2H), 1.84-2.05 (m, 4H), 2.73 (t, ³J= 6.92 Hz, 2H), 5.23-5.36 (br, m, 2H), 6.08 (br, s, 2H), 7.28 (dd, ³J_{AB}= 7.04 Hz, ⁴J= 3.05 Hz, 3H), 7.56 (dd, 3JAB= 7.04 Hz, 4J= 3.05 Hz), 7.65 (d, ³J_{AB}= 7.09 Hz, 2H), 9.33 (³J_{AB}= 7.09 Hz, 2H).

### 1-(4'-Phenylbutyl)-4-nonadecyl pyridinium Chloride

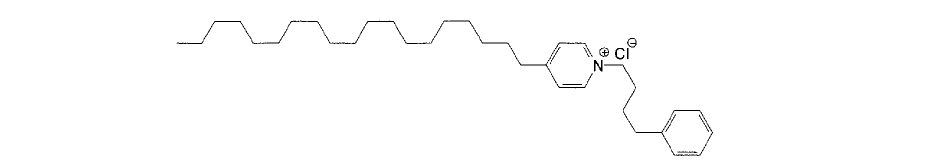

¹H NMR (DMSO-d₆): δ 0.88 (t, ³J= 5.67 Hz, 3H), 1.09-1.40 (m, 32H), 1.63 (m, 4H), 1.97 (m, 2H), 2.61 (t, ³J= 7.02 Hz, 2H), 2.77 (t, ³J= 8.06 Hz, 2H), 4.91 (br, t, J=6.97 Hz, 2H), 7.04-7.27 (m, 5H), 7.69 (d, ³J_{AB}= 7.04 Hz, 2H), 9.27 (d, ³J_{AB}= 7.04 Hz, 2H).

### 1-(4''-Phenylbutyl)-4-(cis-10')-nonadecenyl pyridinium Chloride

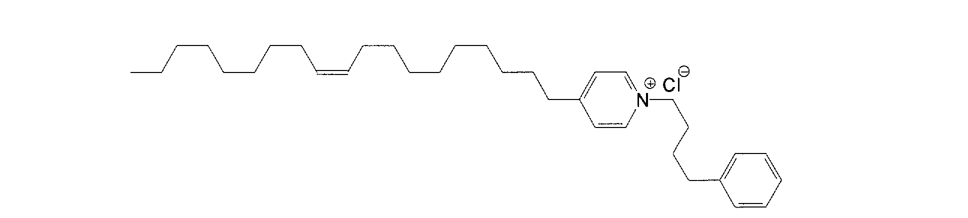

¹H NMR (CD₃OD): δ 0.89 (t, ³J= 7.01 Hz, 3H), 1.09-1.40 (m, 26H), 1.58-1.84 (m, 4H), 1.89-2.13 (m, 4H), 2.68 (t, 3J= 6.91 Hz, 2H), 2.97 (t, 3J= 7.99 Hz, 2H0, 4.56 (br, m, 2H), 5.23-5.36 (m, 2H), 7.09-7.36 (m, 5H), 7.92 (d, ³J_{AB}= 7.07 Hz, 2H), 8.79 (d, ³J_{AB}= 7.07 Hz, 2H).

### 1-(6'-Phenylhexyl)-4-nonadecyl pyridinium Chloride

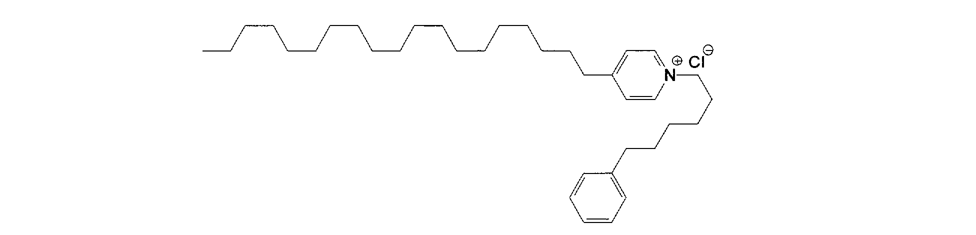

¹H NMR (CD₃OD): δ 0.87 (t, ³J= 6.03 Hz, 3H), 1.3-1.44 (m, 34H), 1.49-1.76 (m, 4H), 1.89-2.09 (m, 4H), 2.56 (t, ³J= 7.03 Hz, 2H), 2.84 (t, ³J= 7.01 Hz, 2H), 4.93 (br, m, 2H), 7.09-7.31 (m, 5H), 7.80 (d, ³J_{AB}= 7.05 Hz, 2H), 9.34 (d, ³J_{AB}= 7.05 Hz, 2H).

### 1-(6''-Phenylhexyl)-4-(cis-10')-nonadecenyl pyridinium Bromide

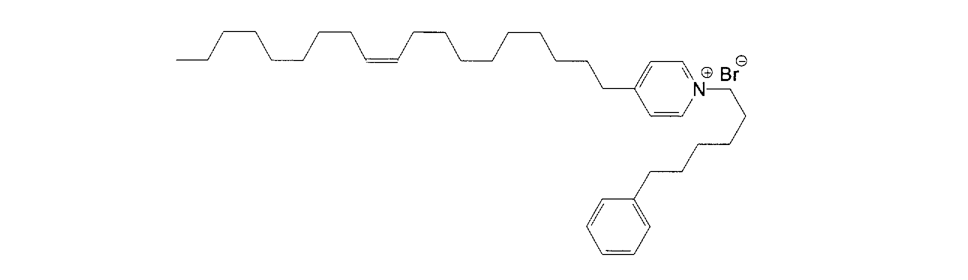

¹H NMR (CD₃OD): δ 0.81 (t, ³J= 6.02 Hz, 3H), 1.13-1.40 (br, m, 30H), 1.56 (t, ³J= 8.02 Hz, 2H), 1.64 (t, 3J= 7.04 Hz, 2H), 1.84-2.04 (br, m, 4H), 2.52 (t, ³J= 8.02 Hz, 2H), 2.86 (t, ³J= 7.94 Hz, 2H), 4.46 (t, ³J= 6.68 Hz, 2H), 5.22-5.36 (m, 2H), 7.01-7.18 (m, 5H), 7.86 (d, ³J_{AB}= 7.02 Hz, 2H), 8.73 (d, ³J_{AB}= 7.02 Hz).

### 1,1'-bis-(1''',4''''-butyl)-4-(cis-10'')-nonadecenyl pyridinium Chloride

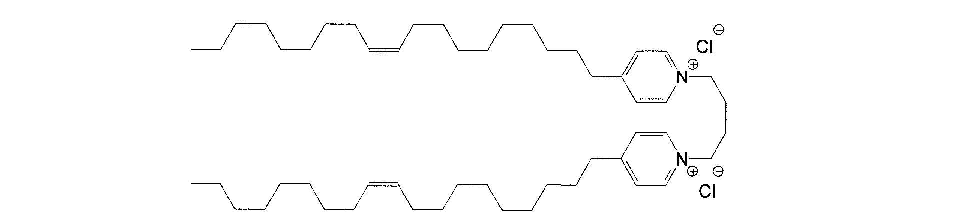

¹H NMR (CDCl₃): δ 0.83 (t, ³J= 7.00 Hz, 6H), 1.08-1.38 (m, 44H), 1.50-1.79 (m, 8H), 1.96-2.05 (m, 8H), 2.30-2.48 (m, 4H), 2.90 (m, 8H), 5.02 (br, m, 4H), 5.25-5.41 (m, 4H), 7.76 (d, ³J_{AB}= 8.67 Hz, 4H), 9.80 (d, ³J_{AB}= 8.67 Hz, 4H).

### 1,1'-bis-(1''',6''''-hexyl)-4-(cis-10'')-nonadecenyl pyridinium Chloride

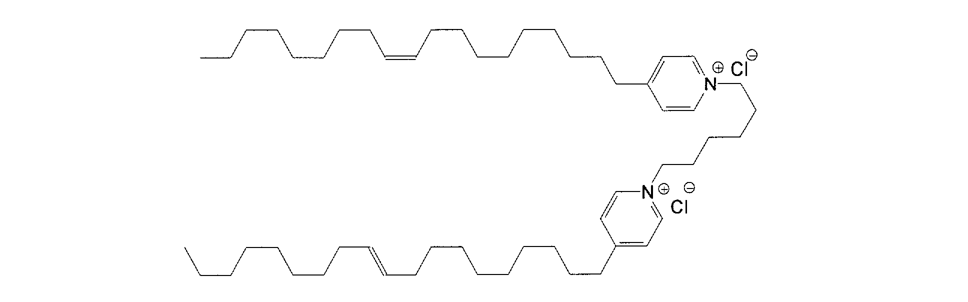

¹H NMR (CDCl₃): δ 0.83 (t, ³J= 7.01 Hz, 6H), 1.08-1.40 (m, 46H), 1.42-1.79 (m, 8H), 1.96-2.09 (m, 8H), 2.11-2.24 (m, 4H), 2.93 (m, 8H), 4.97 (br, m, 4H), 5.21-5.39 (m, 4H), 7.79 (d, ³J_{AB}= 6.35 Hz, 4H), 9.81 (d, ³J_{AB}= 6.35 Hz, 4H).

### 1-Methyl-4-(10'-cis-nonadecenyl) pyridinium Chloride

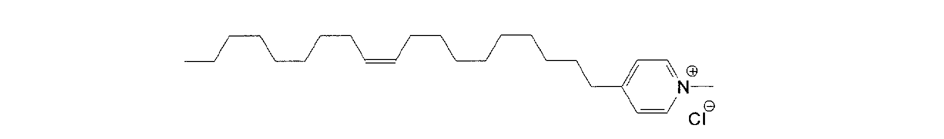

¹H NMR (CDCl₃): δ 0.82 (t, ³J= 7.81 Hz, 3H), 1.06-1.52 (m, 26H), 1.60-1.92 (m's, 4H), 4.89 (t, ³J= 6.62 Hz, 2H), 4.92 (br, m, 3H), 5.30-5.56 (m, 4H), 7.98 (d, ³J_{AB}= 6.87 Hz, 2H), 9.45 (d, ³J_{AB}= 6.87 Hz, 2H).

### 1-(9 '-cis-octadecenyl)-4-(10"-cis-nonadecenyl) pyridinium Chloride

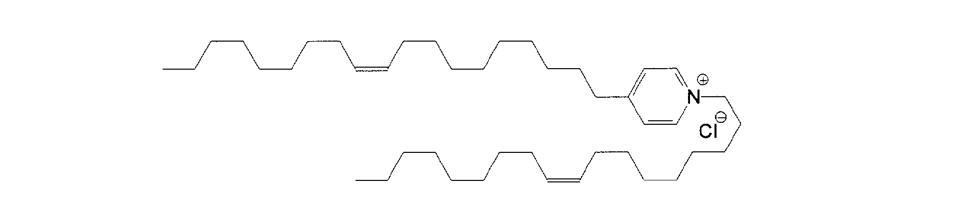

¹H NMR (CDCl₃): δ 0.85 (t, ³J= 6.80 Hz, 6H), 1.20-1.45 (br, m, 46H), 1.50-1.80 (br, m, 4H), 1.90-2.10 (br, m, 8H), 2.87 (t, ³J= 8.11 Hz, 2H), 4.98 (t, ³J= 7.31 Hz, 2H), 5.32-5.54 (m, 4H), 7.94 (d, ³J_{AB}= 6.22 Hz, 2H), 9.43 (d, ³J_{AB}= 6.22 Hz, 2H).

### 1-(hexadecyl)-4-(10''-cis-nonadecenyl) pyridinium Chloride

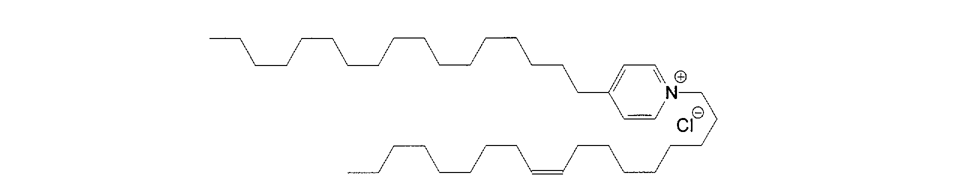

¹H NMR (CDCl₃): δ 0.82-0.94 (t's, 6H), 1.20-1.45 (br, m, 46H), 1.50-1.85 (br, m, 4H), 1.91-2.14 (br, m, 8H), 2.84 (t, 2H), 4.88 (t, ³J= 7.30 Hz, 2H), 5.30-5.45 (m, 2H), 7.87 (d, ³J_{AB}= 6.34 Hz, 2H), 9.56 (d, ³J_{AB}= 6.34 Hz, 2H).

### 1-(9'-cis-nonadecenyl)-4-(-carbo-(9"-cis-nonadecenyloxy)) pyridinium Chloride

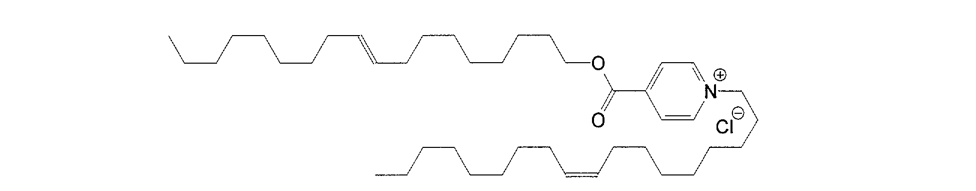

¹H NMR (CDCl₃): δ 0.82 (dt, ³J= 6.96 Hz, 6H), 1.20-1.45 (br, m, 22H), 1.48-1.54 (br, m, 20H), 1.72-1.83 (m, 2H), 1.90-2.08 (br, m, 12 H), 4.67 (t, ³J= 6.87 Hz, 2H), 5.09 (br, t, ³J= 7.24 Hz, 2H), 5.20-5.45 (m, 4H), 8.58 (d, ³J_{AB}= 5.81 Hz, 2H), 9.61 ((d, ³J_{AB}= 5.81 Hz, 2H).

### 1-(9'-cis-nonadecenyl)-4-(-carbo-(hexadecyloxy)) pyridinium Chloride

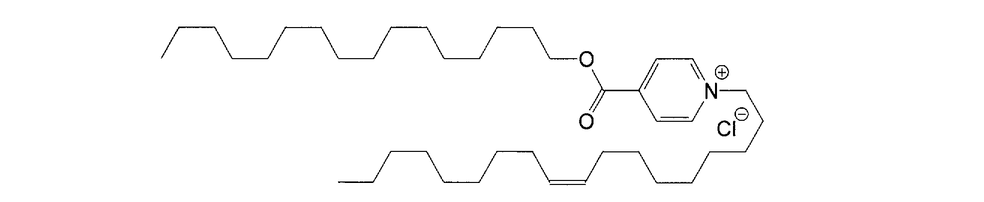

¹H NMR (CDCl₃): δ 0.88-0.94 (t's, 6H), 1.20-1.45 (br, m, 26H), 1.48-1.54 (br, m, 16H), 1.70-1.90 (m, 4H), 1.92-2.10 (br, m, 12H), 4.64 (t, ³J= 6.81 Hz, 2H), 5.05 (br, t, ³J= 7.20 Hz, 2H), 8.52 (d, ³J_{AB}= 6.21 Hz, 2H), 9.56 (d, ³J_{AB}= 6.21 Hz, 2H).

### 1-Methyl-4-(-carbo-(9'-cis-nonadecenyloxy)) pyridinium Chloride

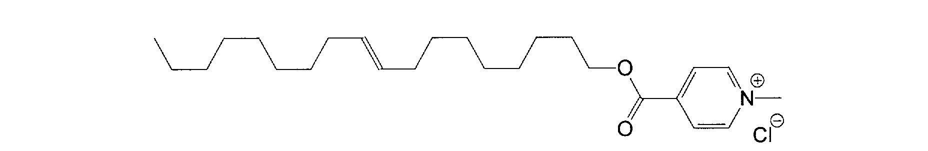

¹H NMR (CDCl₃): δ 0.86 (t, ³J= 6.05 Hz, 3H), 1.08-1.37 (m, 22H), 1.52-1.76 (m, 2H), 1.82-2.04 (m, 4H), 4.45 (t, ³J= 6.89 Hz, 2H), 4.79 (br, m, 3H), 5.21-5.40 (m, 2H), 8.46 (d, ³J_{AB}= 6.68 Hz, 2H), 9.45 (d, ³J_{AB}= 6.68 Hz, 2H).

### 1-Methyl-4-(-carbo-(hexadecyloxy)) pyridinium Chloride

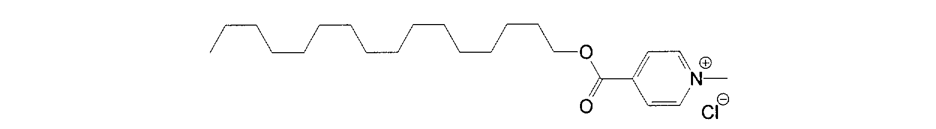

¹H NMR (CDCl₃): δ 0.90 (t, ³J= 6.51 Hz, 3H), 1.18-1.51 (m, 22H), 1.60-1.82 (m, 6H), 4.67 (t, ³J= 6.48 Hz, 2H), 4.81 (br, m, 3H), 8.46 (d, ³J_{AB}= 5.61 Hz, 2H), 9.49 (d, ³J_{AB}= 5.61 Hz, 2H).

### Transfection protocol

Transfections are carried out following standard protocols as is described in EP 0755924. In numerous publications variations of transfection protocols are given. A person acquainted with the field of non-viral transfections, in particular transfections using cationic amphiphiles will be able to use the cationic amphiphiles of the invention in transfection methods known per se. In standard experiments COS-7 cells are transfected with plasmid DNA containing the β-galactosidase reporter gene. Upon successful transfection the cell produces the enzyme β-galactosidase whose activity can be easily measured. Other plasmids comprising alternative reporter genes such as the gene encoding Green Fluoerscent Protein or Chloroamphenicol Acetyl Transferase are suited as well. For analyzing stable transfection a plasmid containing the neomycin resistance gene can be used. Toxicity of the cationic amphiphiles towards the cells can be monitored qualitatively and quantitatively by visual inspection of the cells and by a protein determination.

### Transfection with 1-(9'-cis-octadecenyl)-4-(10"-cis-nonadecenyl) pyridinium Chloride (cationic amphiphile of the formula in claim 1 in which R1 = R2 = nC₁₈H₃₅, A = CH₂ and A is attached in the para position, X = Cl⁻)

A solution of cationic amphiphile in a 50/50 molar ratio with DOPE in chloroform was dried under a stream of nitrogen. The residual solvent was removed under vacuum. The lipid film was hydrated in water at room temperature and sonicated to clarity in a bath sonicator immediately before use.

A 7.1 kb pair plasmid containing the *E.coli* β-galactosidase gene under control of the cytomegalovirus immediate early gene promoter/enhancer (pCMV β-gal Clontech, Palo Alto, CA, USA). DNA was isolated from *E.coli* using a Qiagen Plasmid Kit (QIAGEN Inc, USA). The plasmid concentration was determined by measuring the absoption at 260 nm using the relation 1.0 OD = 50 µg/ml. The OD₂₆₀/OD₂₈₀ value was 1.95.

COS-7 cells were cultured in Dulbecco's Modified Eagle Medium (DMEM Gibco The Netherlands) containing 7% of fetal calf serum, 2 mM L-Glutamine, 100 units/ml of penicilin and 100 mg/ml of streptomycin at 37 °C in CO₂/air (1:19).

Cells (1x 10⁵ cells/well) were seeded in 12-well plates and were allowed to grow overnight.

The complex of cationic amphiphile/DOPE (1:1) with pCMV β-gal was prepared at the charge ratio 2.5:1 (15 nmol lipid and 1 µg DNA), in 100µl 10 mM HEPES (pH=7.4, 150 mM NaCl). After 10-15 min incubation at room temperature, the resulting complex was diluted in 1 ml medium (DMEM) and 0.5 ml of the mixture was added to the cells and incubated for 4 h at 37°C. The β-galactosidase assay was performed 48 later using CPRG-substrate.

Control wells underwent identical steps except that no cationic amphiphile was added. β-galactosidase activity was observed whereas in control wells no activity was detected. The β-galactosidase activity obtained using the cationic amphiphile according to the invention was substantially higher than the activity obtained with Lipofectin™ (Felgner et al. Proc. Natl. Acad. Sci USA, 1987, 84, 7413-7417). The cationic amphiphile displayed no toxicity towards the cells as was analyzed in a protein determination and compared with the reference value of control wells.

### Transfection protocol in the absence of helper lipid with complexation of cationic amphiphile and DNA in small volume of HBS followed by dilution in medium

In a comparative experiment the following cationic amphiphiles were used for transfection:
A: a compound having the formula of claim 1 in which R2-A is (C₁₆H₃₃)₂CH and A is attached in the para position, X is Cl⁻ and R1 is CH₃
B: a compound having the formula of claim 1 in which R2-A is (C₁₆H₃₃)₂CH and A is attached in the para position, X is Cl⁻ and R1 is (CH₂)₄NH₃⁺.

Solutions of A, B and plasmid DNA in HBS (10 mM HEPES, 150 mM NaCl, pH 7.4) were prepared. The concentrations used were for A 7.5 and 15 nmol per 50 µl, for B 7.5 nmol per 50 µl and for DNA 1 µg per 50 µl.

For each concentration of amphiphile 50 µl was mixed with 50 µl of DNA solution. After a short incubation time of 15 minutes this was diluted with medium to a volume of 1 ml.

COS-7 cells (1x 10⁵ cells/well) were seeded in 12-well plates and were allowed to grow overnight. 0.5 ml of the mixture obtained above was added to one well. The cells were incubated at 37°C for 4 hours followed by a β-galactosidase activity determination after 48 hours.

In a control well no β-galactosidase activity was observed.

For A, β-galactosidase activity was observed in approximately equal amounts for both concentrations.

For B a β-galactosidase activity was observed which was approximately 25 to 30 times higher than for A.

## Claims

1. Cationic amphiphiles having an aromatic ring comprising a nitrogen atom according to the following formula 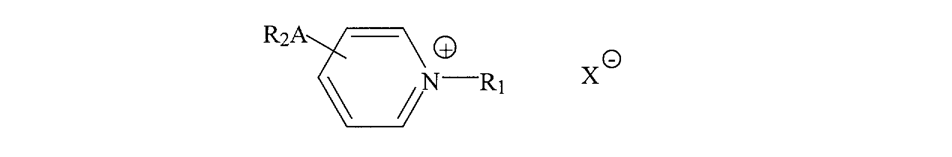 in which:
R1 is selected from the group consisting of:
a branched or linear (C6-C24) carbon chain optionally interrupted by one or more heteroatoms, optionally containing one or more functional groups, optionally containing one or more double or triple carbon-carbon bonds or combinations of double and triple carbon-carbon bonds, optionally being substituted or combinations thereof and
R3-Ar-A-R2 in which R3 is a C2-C10 carbon chain, Ar is an aromatic or a heteroaromatic ring optionally to which relative to R3 in the ortho-, meta-, or para-position A-R2 is attached,
A is attached to the aromatic ring in the ortho-, meta- or para-position relative to the nitrogen atom in the aromatic ring,
A is selected from the group consisting of CH₂, O, S, ,S-S, NH, OC=O, O=CO, SC=O, O=CS, NHC=O, O=CNH, CH=N, N=CH, phosphate, alkylphosphate and phosphonate R2 represents a branched or linear (C6-C24) carbon chain optionally interrupted by one or more heteroatoms, optionally containing one or more functional groups, optionally containing one or more double or triple carbon-carbon bonds or combinations of double and triple carbon-carbon bonds, optionally being substituted or combinations thereof X⁻ is a physiologically acceptable anion.

2. Cationic amphiphile according to claim 1 in which A is CH₂ and is attached in the para-position relative to the nitrogen atom in the aromatic ring.

3. Cationic amphiphile according to claim 1 in which A is OC=O and is attached in the para-position relative to the nitrogen atom in the aromatic ring.

4. Cationic amphiphile according to claim 1 in which and A is OC=O and is attached in the meta-position relative to the nitrogen atom in the aromatic ring.

5. Cationic amphiphile according to any of claims 2-4 in which R1 is a carbon chain selected from the group consisting of C16, C18, C20 and C22 carbon atoms, optionally containing one or more double or triple carbon-carbon bonds or combinations thereof and R2 is selected from the group consisting of C14, C16 and C18 carbon atoms, optionally containing one or more double or triple carbon-carbon bonds or combinations thereof.

6. Cationic amphiphile according to claim 1 in which A is CH₂ and is attached in the para-position relative to the nitrogen atom in the aromatic ring and R2 is R4C=OO in which R4 is a C5-C23 carbon chain.

7. Cationic amphiphile according to claim 6 in which R1 is a carbon chain selected from the group consisting of C16, C18, C20 and C22 carbon atoms, optionally containing one or more double or triple carbon-carbon bonds or combinations thereof and R2 is selected from the group consisting of C11, C13, C15 and C17 carbon atoms, optionally containing one or more double or triple carbon-carbon bonds or combinations thereof.

8. Cationic amphiphile according to any of claims 1-7 in which R1 is longer than R2.

9. Composition comprising a cationic amphiphile according to any of claims 1-8 and a biologically active compound.

10. Composition according to claim 9 in which the biologically active compound is DNA, RNA or an (antisense-)oligonucleotide.

11. Composition according to claim 9 or 10 further comprising a pharmaceutically acceptable carrier.

12. Method for the introduction of biologically active compounds into eukaryotic cells said method comprising the steps of mixing a cationic amphiphile according to claim 1 with a biologically active compound and bringing the mixture thus obtained into contact with the cells to be transfected.

13. Method for the preparation of a cationic amphiphile having a nitrogen atom containing aromatic ring and a group attached to said nitrogen atom in which the step of introducing a group on the nitrogen atom is carried out in a microwave oven.

14. Method according to claim 13 for the introduction of R1 in a cationic amphiphile according to claim 1.

15. Method for the introduction of biologically active compounds into eukaryotic cells comprising the steps of first mixing a cationic amphiphile having an aromatic ring comprising at least one nitrogen atom in the absence of helper lipid with a biologically active compound in a small volume of saline buffer and then diluting the resulting complex in medium and bringing the mixture thus obtained into contact with the cells to be transfected.

16. Method according to claim 15 in which the biologically active compound is DNA, RNA or an (antisense-)oligonuceotide.

17. Method according to claim 15 or 16 in which the cationic amphiphile comprises additional positive charge in the vicinity of the nitrogen atom containing aromatic ring, in particular the cationic amphiphile comprises one or more ammonium groups.
